# EUROPEAN PATENT SPECIFICATION

(11) **EP 4 021 357 B1**
(45) Date of publication and mention of the grant of the patent: **11.09.2024**
(21) Application number: 20857115.8
(22) Date of filing: 26.08.2020
(51) Int. Cl.: A61F 5/56

(54) **ADJUSTMENT MEANS FOR A MANDIBULAR ADVANCEMENT DEVICE**
VERSTELLVORRICHTUNG FÜR EINE MANDIBULÄRE VORSCHUBVORRICHTUNG
MOYEN DE RÉGLAGE POUR UN DISPOSITIF D'AVANCEMENT MANDIBULAIRE

(30) Priority: 26.08.2019 AU 2019903104
(43) Date of publication of application: 06.07.2022
(73) Proprietor: Bioanalytics Holdings Limited, Melbourne, Victoria 3000 (AU)
(72) Inventor: MORGAN, Owen, Kirribilli New South Wales 2061 (AU); YAN, Guoping, Noble Park Victoria 3174 (AU); SASSE, Anthony, Kew Victoria 3101 (AU)
(74) Representative: Potter Clarkson
(86) International application number: PCT/AU2020/000087
(87) International publication number: WO 2021/035277

(56) References cited:
- WO-A1-2019/071291
- WO-A1-2019/071291
- US-A1- 2008 035 157
- US-A1- 2012 199 136
- US-A1- 2013 295 514
- US-A1- 2015 272 773
- US-A1- 2015 272 773
- US-A1- 2019 125 574
- US-B2- 8 783 260

## Description

### Field of the Invention

This invention relates to mandibular advancement devices, particularly to devices worn by a person to prevent obstructive sleep apnoea, and in particular to adjustment means for the intraoral portion of that device.

### Background of the Invention

A mandibular advancement device of the type referred to in this specification is already disclosed in WO 2019/071291 A1, which discloses the preamble of claim 1, and WO 2006/072147. The mandibular advancement device disclosed has both intraoral and extraoral portions. The present invention is mainly concerned with the intraoral portion of the device.

Due to wide variations in the size and shape of the oral cavity of users of these kinds of devices, and to keep manufacturing costs low, these devices are typically manufactured in three broad sizes, such as small, medium and large. However, there is a need for further adjustment within each of these broad sizes to maximise comfort and fit for each particular user, and also to maximise the effectiveness of the device.

One of the main problems surrounding the fit and comfort of the device is getting the engagement of the posterior maxillary abutment surfaces of each of the respective arms of the intraoral portion of the device in the correct position that conforms with the wearer's posterior maxillary characteristics. The posterior maxillary abutment surfaces are meant to loosely engage with the molars or premolars of the wearer and to resist any lateral movement of the device inside the oral cavity of the wearer. It is important that the device does not impose sufficient lateral force to the wearer's teeth so that tooth displacement may occur over time. Also, the imposition of unnecessary force will significantly negatively impact the wearer's comfort when wearing the device and may reduce the device's use and performance.

It is therefore an object of the present invention to provide adjustment means for the intraoral portion of a mandibular advancement device that at least mitigates some of the aforementioned problems.

WO 2019/071291 discloses a method of monitoring the status of a body of a person during sleep, said method comprising: retaining in or adjacent the mouth or nose of said person an electronic detector which detects indications of the state of said body, wirelessly transmitting a first data stream representing said indications to a data receiving means, transmitting said first data stream from said data receiving means to a data processing means, processing said first data stream within said data processing means to produce a second data stream representing said indications, or conclusion drawn from said indications, and displaying a visual representation of said indications or conclusions.

### Summary of the Invention

According to a first aspect, the present invention provides a mandibular adjustment device according to claim 1.

Preferably, the lower plate includes a threaded hole on each side, adjacent a respective arm, so that the threaded hole extends from the respective arm towards the edge of the plate. Each hole is adapted to receive a screw that is able to be screwed into the threaded hole, and when the screw has been sufficiently screwed, the inner end of the screw contacts its respective arm whereupon further turning of the screw forces the arm to move relative to its opposite arm so that the position of the moved arm's posterior intraoral maxillary abutment surface is moved to a new position.

Preferably, each threaded hole in the bottom plate is recessed back from the outer edge and the opening of the threaded hole is respectively located within a vertical slot.

Preferably, the upper plate is adapted to overlay the bottom plate and join together with the bottom plate to form the body portion and encapsulate a portion of each of the arms within the body portion. The upper plate includes side tabs that extend substantially vertically down and are aligned with and adapted to be inserted into, and conform with the shape of, the vertical slot, thereby overlaying the entrance to the threaded hole and screw and forming a body portion with a substantially smooth outer surface.

Preferably, each side tab includes an aperture that is positioned so that it aligns with, and is concentric with, the threaded hole and the screw.

Preferably, each aperture is sized so that an adjustment tool, such as a screw driver or an Allen key is insertable, and is thereby able to engage with and act upon the screw, but is too small to enable the screw to be withdrawn through the aperture, thereby making the tab act as an abutment that prevents the screw from falling out of the body portion.

According to a second embodiment, the adjustment means are either electromechanical, or magnetic, thereby removing the need for an aperture on either the top or bottom plate, allowing the body to have a substantially continuous surface, allowing easier and more effective cleaning and sterilisation.

Preferably, each arm is integrally formed in either the top or bottom plate and fabricated out of a suitable elastomeric material.

Preferably, each arm is naturally biased with respect to the other with a maximum spread that the arms naturally rest at when not acted on by an external force, such as the impingement of the screw as it is operated on by the adjustment tool.

According to a third embodiment of the present invention, each arm is fabricated separately to either the top or bottom plate, and either the top or bottom plate includes a post portion that is profiled to resist rotation around the post, and the anterior end of each arm includes a hole that has a complimentary shape with, and is adapted to receive, the post portion within said hole, and this position is the natural rest position for each arm when the arm is not acted upon by an external force, such as the impingement of the screw as it is operated on by the adjustment tool.

According to a fourth embodiment of the present invention, each arm is fabricated separately to either the top plate or the bottom plate, and either the top plate or bottom plate includes a post portion with a circular profile, and the anterior end of each arm includes a hole that is adapted to receive the post portion and allow the arm to freely rotate about its respective post portion, and the body portion includes separate biasing means that act upon each respective arm and hold them at their maximum spread position when each arm is not acted upon by an external force, such as the impingement of the screw as it is operated on by the adjustment tool.

According to a fifth embodiment of the present invention the biasing means is a coil spring.

According to a sixth embodiment of the present invention, the biasing means is a leaf spring.

According to a seventh embodiment of the present invention, the biasing means is an elastomeric tab that is included in either the top plate or the bottom plate, and each said elastomeric tab is adapted to impinge against a respective arm.

Preferably, either the top or bottom plate includes an aperture, or window, that is associated with measurement increment indicators, and said measurement increments indicate the current position of each arm as an arm is forced to a new position under the influence of the grub screw.

Optionally, the body portion includes electronic sensor means that are adapted to sense the current position of each arm and feed that position data to wireless transmission means such as a Bluetooth transmitter.

Preferably, the data transmitted by the wireless transmission means are processed by an app on a portable computing device, such as a smart phone or tablet.

In another aspect, the present invention is a method of adjusting a mandibular advancement device in order to improve the fit and comfort of the wearer of said device, the method including the steps of:
a. selecting an appropriate device from a selection of small, medium and large size devices; and
b. inserting the device into the wearer's mouth; and
c. determining the fit and comfort of the device by bracing each intraoral maxillary abutment surface against an extramaxillary anterior maxillary abutment surface; and
d. receiving feedback from the wearer on the fit and comfort; and
e. using an appropriate tool to insert into an adjustment hole, if necessary, to turn the grub screw and force each respective arm to change position and thereby causing each posterior intraoral maxillary abutment surface to change position to provide a custom fit that best suits the wearer's mandibular.

Preferably, the method further includes the step of using the aperture, or window, and its associated measurement increment indicators to take note of the most comfortable setting for the particular wearer.

Optionally, the method further including the step of using the sensor means and wireless transmission means to transmit the position of each arm in an app on a portable computing device, such as a smart phone, and using the app to associate the optimal position of each arm to the wearer.

### Brief Description of the Drawings

Examples of the invention will now be described with reference to the accompanying drawings in which:
Figure 1 is an isometric view of the top side of the bottom plate.
Figure 2 is another isometric view of the top of the bottom plate including a pair of grub screws.
Figure 3 is an exploded view of the top and bottom plates.
Figure 4 is an isometric view of the top and bottom plates joined together to form the body portion.
Figure 5 shows the assembled substantially intraoral portion of the mandibular advancement device.
Figure 6 shows a further embodiment of the invention so that the position of each arm can be measured.
Figure 7 shows another embodiment of the invention where electronic sensors are included that sense the respective position of each arm and optionally transmit that data to an app on a portable computing device, such as a smart phone.
Figures 8 (a) and (b) show another preferred embodiment of the current invention where each arm is fabricated separately to the body portion and each arm is able to be placed upon a respective stake prefabricated into the bottom plate and is free to rotate about its respective stake, and the use of a natural leaf spring to hold each arm apart.
Figures 9 (a) and (b) show yet another preferred embodiment of the current invention where each arm is fabricated separately and is each able to be placed upon a respective stake that has been prefabricated into the bottom plate, however each arm is unable to rotate about its respective stake and thereby held in its maximum spread position.
Figures 10 (a) and (b) show another preferred embodiment of the invention using a pair of conventional coil springs to hold each respective arm in its maximum spread position.

### Detailed Description of the Preferred Embodiments

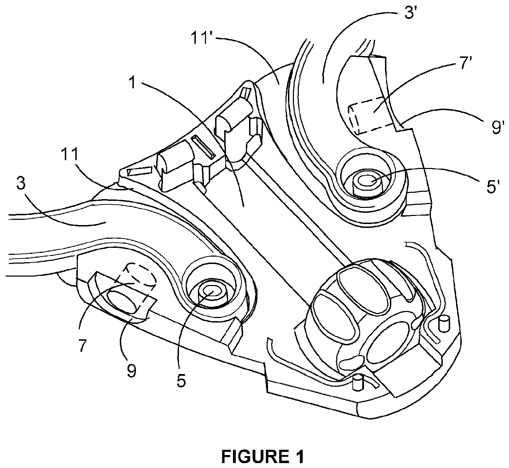

Turning firstly to Figure 1, where we are shown an isometric view of the top of the bottom plate 1. The bottom plate 1 is a part of the body portion of the intraoral portion of a mandibular advancement device. The bottom plate 1 includes a pair of arms 3 and 3' respectively. Each of the arms 3 and 3' recede into the wearer's oral cavity. In this preferred embodiment, each arm is integrally formed in the bottom plate at 5 and 5' respectively. Each of the arms 3 and 3' are fabricated from a suitably resilient material that will elastically yield under the influence of an external force. Each arm rests at its maximum spread position with respect to its opposite arm, when not acted upon by an external force.

Adjacent to each arm is a threaded hole 7 and 7' respectively. Each hole is reset back from the edge of the bottom plate 1 and each is substantially centrally located in a respective slot 9 and 9'. Each threaded hole 7 and 7' is open at its innermost end.

Each arm 3 and 3' respectively also has a gap 11 and 11' into which each arm is able to move when acted upon by an external force in order to bring the arms closer together.

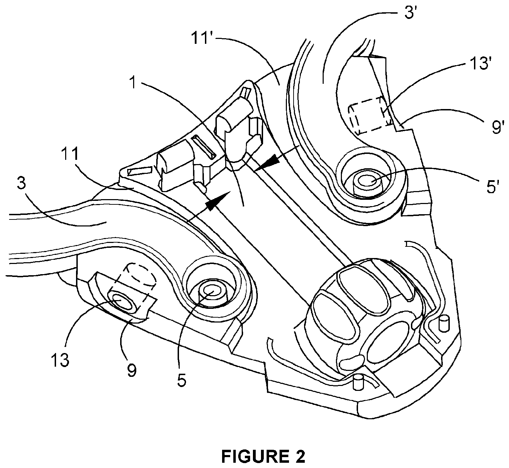

Turning to Figure 2, we are shown that a pair of grub screws 13 and 13' are adapted to be screwed into each respective threaded hole. When a tool such as a screw driver, or Allen key or the like is used, the grub screws may be turned. As each is turned, eventually the innermost end of the screw impinges upon the outermost side of its respective arm. This forces the arm to yield under the force applied by the screw. This causes the respective arm to move in the direction indicated by the arrows.

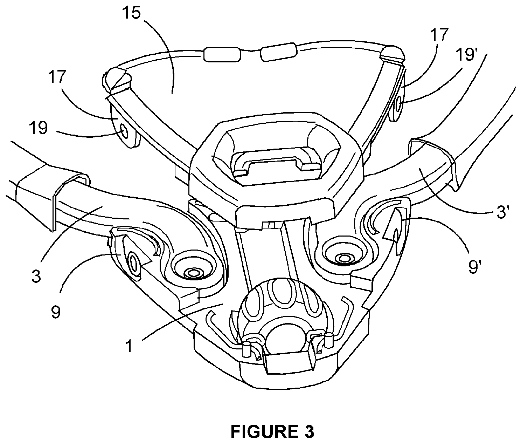

Turning to Figure 3, we are shown the top plate 15 ready to be attached to the bottom plate 1. The top plate 15 includes a pair of downwardly extending tabs 17 and 17' respectively. The size and shape of each of the tabs is selected so that when the top and bottom plates are connected, the tabs fit flush inside the respective slots 9 and 9'. Each tab 17 and 17' includes a small aperture 19 and 19'. These apertures are adapted to be concentric with the hole and grub screws when the body portion is assembled. The aperture is sized so that a tool may pass through the aperture and engage the head of the grub screw, thereby enabling it to be turned in order to adjust the position of each arm. It is also sized so that the aperture is too small for the grub screw to be able to withdraw back through it if the screw is unwound within the threaded hole. Each respective tab 17 and 17' thereby acts as an abutment that prevents it's respective grub screw from falling out of the body portion.

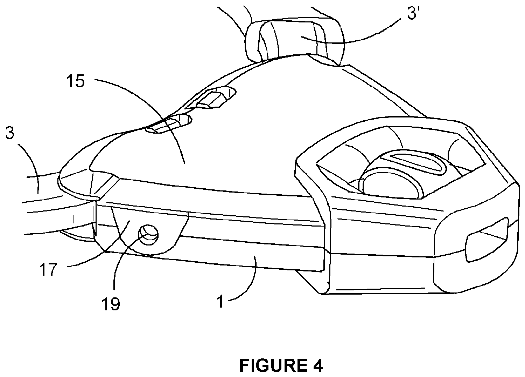

In Figure 4, the body portion is assembled and illustrates how the tab 17 in the top plate 15 fits flush with the slot in the bottom plate 1.

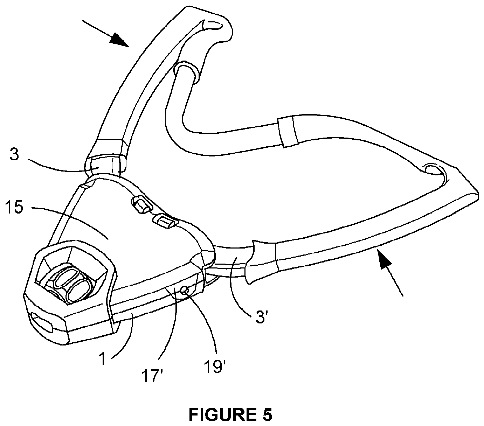

Figure 5 shows the body portion comprising top plate 15 and bottom plate 1 assembled with other intraoral components of the mandibular advancement device. Adjustment of each respective grub screw by inserting a tool through aperture 19 and 19' causes the arms 3 and 3' to move closer together as shown by the arrows. In this embodiment, due to the natural resilience of the material from which the arms are fabricated, unscrewing the grub screw causes its respective arm to return to its normal rest position.

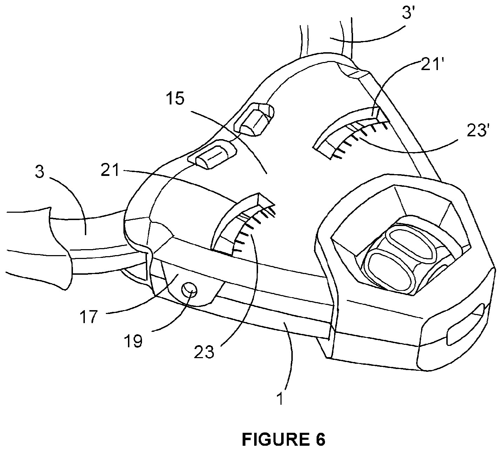

Figure 6 shows an optional feature on the top plate 15. In this embodiment of the invention, a pair of windows 21 and 21'. Each window includes measurement graduations 23 and 23'. These offer the user a visual aid in determining the position of each arm 3 and 3'. The user can then determine the best arm position for that particular wearer of the device. This will particularly assist a user to setup subsequent devices for a particular wearer and manually set the arms into the initial best position for that particular person.

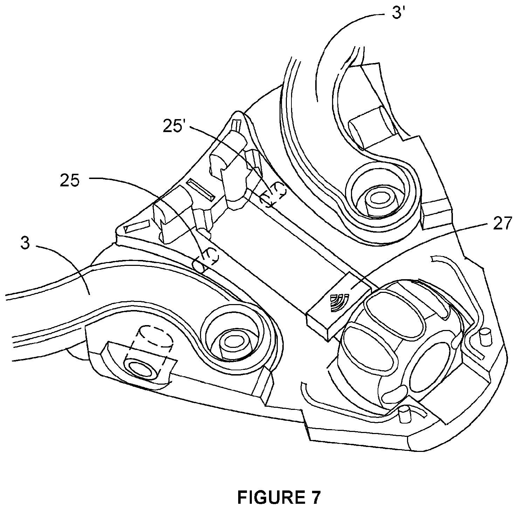

Figure 7 shows another preferred embodiment of the invention. In this view, the body portion is fitted with electronic sensors 25 and 25 '. These sensors electronically sense the position of each arm 3 and 3'. The position data is then optionally transmitted via wireless means 27 to a portable computing device such as a smartphone. The smartphone has an app installed that links the position data to the user's account and logs the selected position of each arm that the wearer of the device finds to be the most comfortable. This data can then be used in the future when setting up ancillary or replacement devices for that particular wearer.

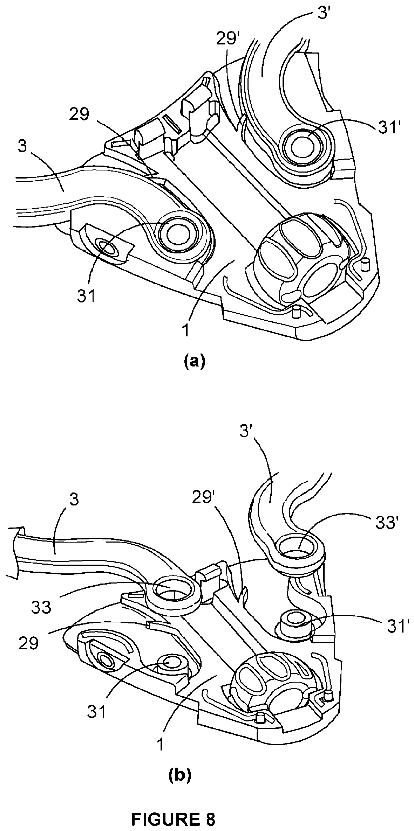

Figures 8 (a) and (b) show a preferred embodiment of the invention where a pair of natural leaf springs 29 and 29' are prefabricated into the bottom plate 1. Each of the arms 3 and 3' are fabricated separately to the bottom plate 1, and each arm includes a circular hole 33 and 33' respectively at its anterior end which is designed to be placed upon a stake 31 and 31' respectively. Each stake also has a matching circular profile. Each arm is then able to rotate about its respective stake, however each arm is biased into its maximum spread position by the force applied upon it by a respective leaf spring.

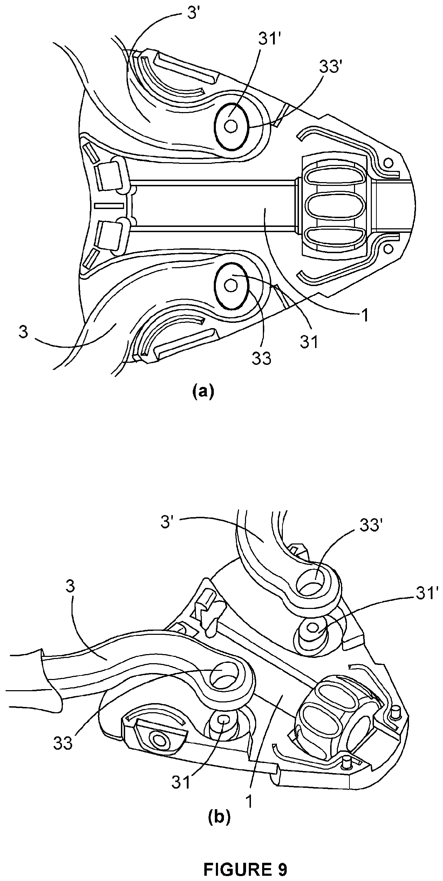

Figures 9 (a) and (b) show another preferred embodiment of the present invention. In this embodiment, each of the arms 3 and 3' are fabricated separately to the bottom plate 1, however each of the holes 33 and 33' are no longer circular in profile. Each of the stakes 31 and 31' that a respective arm is placeable upon has a matching non-circular profile. The shape of the stake and the hole on the arm prevent the arm from rotating about the stake, and this holds each arm in its maximum spread position. Any force applied to a respective arm imposed by the grub screw will cause the arm to move towards its opposite arm. When the force is removed, the arm will move back to its maximum spread position under the influence of its own resilience.

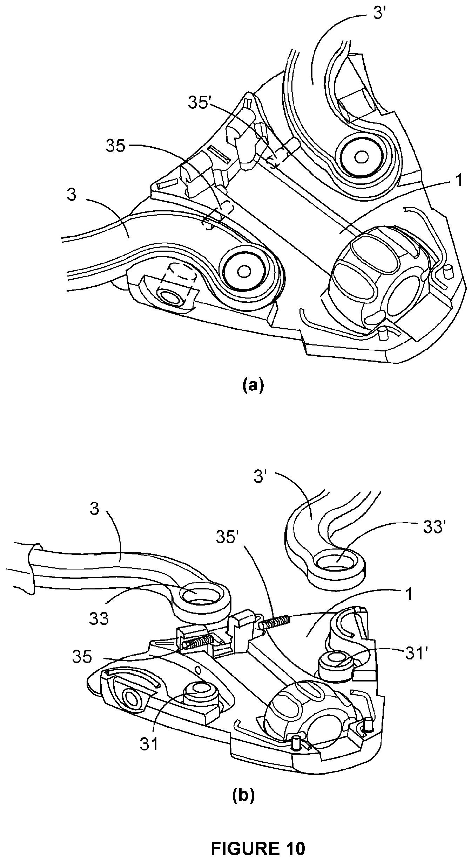

Figures 10 (a) and (b) show another preferred embodiment of the present invention. In this embodiment, each of the arms 3 and 3' are fabricated separately to the bottom plate 1, and each is able to be placed upon a prefabricated stake 31 and 31', and both the stakes and the holes in the arms have a circular profile, so that each arm is able to rotate around its respective stake, however a pair of springs 35 and 35' are included in the assembly, and each spring applies a biasing force to a respective arm that holds the arm in its maximum spread position until acted upon by a respective grub screw.

So, from the aforementioned preferred embodiments, it can be seen that the present invention provides an easy way for a user to setup the intraoral portion of a mandibular advancement device so that it may have improved fit, comfort upon the wearer, and may also provide more efficient function.

While the above description includes the preferred embodiments of the invention, it is to be understood that many variations, alterations, modifications and/or additions may be introduced into the constructions and arrangements of parts previously described without departing from the essential features of the invention. For example, the adjustment means in the preferred embodiment uses a threaded hole and associated grub screw, however other means of adjusting the position of the arm with respect to the other arm are possible, such as elector-mechanical means, or magnetic means. These should be considered within the scope of the present invention. Such means offer the ability to avoid the need for apertures in both the top and bottom plates, thereby making their respective surfaces continuous and thereby easier to clean and to sterilise between uses.

It will be also understood that where the word "comprise", and variations such as "comprises" and "comprising", are used in this specification, unless the context requires otherwise such use is intended to imply the inclusion of a stated feature or features but is not to be taken as excluding the presence of other feature or features.

The reference to any prior art in this specification is not, and should not be taken as, an acknowledgment or any form of suggestion that such prior art forms part of the common general knowledge.

## Claims

1. A mandibular advancement device including:
an intraoral portion including a body portion, the body portion including lower (1) and upper (15) plates, the lower plate (1) being adapted to accommodate a pair of opposed arms (3, 3'), that extend rearwardly and outwardly from the lower plate (1) into an oral cavity when the device is worn, each arm including a posterior intraoral maxillary abutment surface, and
an extraoral portion,
**characterised in that**
the mandibular advancement device includes an adjustment means provided on each side of the lower plate (1), the adjustment means adapted to act upon a respective arm to move the respective arm towards its opposing arm, so that the respective arm's posterior intraoral maxillary abutment surface is moved to a new position.

2. The mandibular advancement device as defined in claim 1 wherein the adjustment means comprises screws (13, 13'), and wherein each side of the lower plate (1) of the device includes a threaded hole (7, 7'), adjacent a respective arm, so that the threaded hole (7, 7') extends from the respective arm towards an edge of the lower plate (1), and wherein each hole (7, 7') is adapted to receive a screw of the adjustment means that is able to be screwed into the threaded hole (7, 7'), and when the screw (13, 13') has been sufficiently screwed into said threaded hole (7, 7'), an inner end of the screw (13, 13') contacts its respective arm whereupon further turning of the screw (13, 13') forces the respective arm to move towards its opposing arm so that the respective arm's posterior intraoral maxillary abutment surface is moved to a new position.

3. The mandibular advancement device as defined in claim 2 wherein:
each threaded hole (7, 7') in the lower plate (1) of the device is recessed back from the edge and an opening of the threaded hole (7, 7') is respectively located within a vertical slot (9, 9'); and optionally
the upper plate (15) of the device is adapted to overlay the lower plate (1) and join together with the lower plate (1) to form the body portion and encapsulate a portion of each of the arms within the body portion.

4. The mandibular advancement device as defined in claim 3 wherein the upper plate (15) of the device includes side tabs (17, 17') that extend substantially vertically down and are aligned with and adapted to be inserted into, and conform with the shape of, the vertical slot (9, 9'), thereby overlaying the opening of the threaded hole (7, 7') and screw (13, 13') and forming the body portion with a substantially smooth outer surface.

5. The mandibular advancement device as defined in claim 4 wherein each side tab (17, 17') of the device includes an aperture that is positioned so that it aligns with, and is concentric with, the threaded hole (7, 7') and the screw (13, 13').

6. The mandibular advancement device as defined in claim 5 wherein each aperture of the device is sized so that an adjustment tool is insertable, and is thereby able to engage with and act upon the screw (13, 13'), but is too small to enable the screw (13, 13') to be withdrawn through the aperture, thereby making the side tab (17, 17') act as an abutment that prevents the screw (13, 13') from falling out of the body portion.

7. The mandibular advancement device as defined in claim 1 wherein each arm (3, 3') of the device is integrally formed in either the upper or lower plate and fabricated out of a suitable elastomeric material.

8. The mandibular advancement device as defined in claim 7 wherein each arm of the device is naturally biased with respect to the other with a maximum spread that the arms naturally rest at when not acted on by the adjustment means.

9. The mandibular advancement device as defined in claim 1 wherein:
each arm of the device is fabricated separately to either the upper or lower plate (1), and either the upper or lower plate (1) includes a respective post portion for each arm that is profiled to resist rotation around the post portion, and an anterior end of each arm includes a hole (33, 33') that has a complimentary shape with, and is adapted to receive, the post portion within said hole (33, 33'), the post portion being received within said hole (33, 33') being a natural rest position for each arm when the arm is not acted upon by the adjustment means; or
each arm of the device is fabricated separately to either the upper plate (15) or the lower plate (1), and either the upper plate (15) or lower plate (1) includes a respective post portion with a circular profile, and an anterior end of each arm includes a hole that is adapted to receive the post portion and allow the arm to freely rotate about its respective post portion, and the body portion includes separate biasing means that act upon each respective arm and hold the arms at their maximum spread position when each arm is not acted upon by the adjustment means.

10. The mandibular advancement device as defined in claim 9 wherein the biasing means of the device is at least one of a coil spring, a leaf spring, or an elastomeric tab that is included in either the upper plate (15) or the lower plate (1), the elastomeric tab being adapted to impinge against a respective arm.

11. The mandibular advancement device as defined in any previous claim wherein either the upper or lower plate (1) of the device includes an aperture, or window, that is associated with measurement increment indicators, and said measurement increment indicators indicate a current position of each arm as the arm is forced to a new position by the adjustment means.

12. The mandibular advancement device as defined in claim 11 wherein the body portion of the device includes electronic sensor means that are adapted to sense the current position of each arm and feed that position data to wireless transmission means such as a Bluetooth transmitter; and optionally
the position data are transmitted by the wireless transmission means and are processed by an app on a portable computing device.

13. A method of adjusting the mandibular advancement device as defined in claim 1, the method including:
a. selecting an appropriate mandibular advancement device from a selection of small, medium and large size mandibular advancement devices;
b. inserting the device into a wearer's mouth;
c. determining a fit and comfort of the device by bracing each intraoral maxillary abutment surface against an extramaxillary anterior maxillary abutment surface;
d. receiving feedback from the wearer on the fit and comfort; and
e. using an appropriate tool to engage the adjustment means to force each respective arm to change position and thereby causing each posterior intraoral maxillary abutment surface to change position to provide a custom fit of the device for the wearer.

14. The method as defined in claim 13 wherein step e. comprises using an appropriate tool to insert into the threaded hole and thereby engaging a screw of the adjustment means and turning it to force each respective arm to change position and thereby causing each posterior intraoral maxillary abutment surface to change position to provide a custom fit of the device for the wearer.

15. The method as defined in claim 13 or claim 14 wherein either the upper or lower plate (1) of the device further includes an aperture, or window, that is associated with measurement increment indicators, and said measurement increment indicators indicate a current position of each arm as the arm is forced to a new position under the influence of the screw, said method further including using the aperture, or window, and its associated measurement increment indicators to take note of the most comfortable setting for the particular wearer, and optionally
wherein the body portion of the device further includes electronic sensor means that are adapted to sense the current position of each arm and feed that position data to wireless transmission means such as a Bluetooth transmitter, and wherein the position data are transmittable by the wireless transmission means and are processable by an app on a portable computing device, said method further including using the sensor means and wireless transmission means to transmit the position data of each arm in an app on a portable computing device to associate the optimal position of each arm to the wearer.

## Patentansprüche

1. Unterkiefervorverlagerungsvorrichtung, umfassend:
einen intraoralen Abschnitt, der einen Körperabschnitt umfasst, wobei der Körperabschnitt untere (1) und obere (15) Platten umfasst, wobei die untere Platte (1) so angepasst ist, dass sie ein Paar gegenüberliegender Arme (3, 3') aufnehmen kann, die sich nach hinten und außen von der unteren Platte (1) in eine Mundhöhle erstrecken, wenn die Vorrichtung getragen wird, wobei jeder Arm eine hintere intraorale Oberkieferanschlagfläche umfasst, und
einen extraoralen Abschnitt,
**dadurch gekennzeichnet, dass**
die Unterkiefervorverlagerungsvorrichtung ein Einstellmittel umfasst, das auf jeder Seite der unteren Platte (1) vorgesehen ist, wobei das Einstellmittel so angepasst ist, dass es auf einen jeweiligen Arm einwirkt, um den jeweiligen Arm in Richtung des gegenüberliegenden Arms zu bewegen, so dass die hintere intraorale Oberkieferanschlagfläche des jeweiligen Arms in eine neue Position bewegt wird.

2. Unterkiefervorverlagerungsvorrichtung gemäß Anspruch 1, wobei das Einstellmittel Schrauben (13, 13') umfasst, und wobei jede Seite der unteren Platte (1) der Vorrichtung ein Gewindeloch (7, 7') neben einem jeweiligen Arm aufweist, so dass das Gewindeloch (7, 7') vom jeweiligen Arm in Richtung einer Kante der unteren Platte (1) verläuft, und wobei jedes Loch (7, 7') dazu geeignet ist, eine Schraube des Einstellmittels aufzunehmen, die in das Gewindeloch (7, 7') eingeschraubt werden kann, und wenn die Schraube (13, 13') ausreichend in das Gewindeloch (7, 7') eingeschraubt wurde, ein inneres Ende der Schraube (13, 13') ihren jeweiligen Arm berührt, woraufhin ein weiteres Drehen der Schraube (13, 13') den jeweiligen Arm dazu zwingt, sich in Richtung seines gegenüberliegenden Arms zu bewegen, so dass die hintere intraorale Oberkiefer-Anschlagfläche des jeweiligen Arms in eine neue Position bewegt wird.

3. Unterkiefervorverlagerungsvorrichtung gemäß Anspruch 2, wobei:
jedes Gewindeloch (7, 7') in der unteren Platte (1) der Vorrichtung von der Kante zurückgesetzt ist und eine Öffnung des Gewindelochs (7, 7') jeweils innerhalb eines vertikalen Schlitzes (9, 9') angeordnet ist; und optional
die obere Platte (15) der Vorrichtung so angepasst ist, dass sie die untere Platte (1) überdeckt und sich mit der unteren Platte (1) verbindet, um den Körperabschnitt zu bilden und einen Abschnitt jedes der Arme innerhalb des Körperabschnitts einzukapseln.

4. Unterkiefervorverlagerungsvorrichtung gemäß Anspruch 3, wobei die obere Platte (15) der Vorrichtung seitliche Laschen (17, 17') aufweist, die im Wesentlichen vertikal nach unten verlaufen, und auf den vertikalen Schlitz (9, 9') ausgerichtet sind und in diesen eingesetzt werden können und sich seiner Form anpassen, wodurch sie die Öffnung des Gewindelochs (7, 7') und der Schraube (13, 13') überdecken und den Körperabschnitt mit einer im Wesentlichen glatten Außenfläche bilden.

5. Unterkiefervorverlagerungsvorrichtung gemäß Anspruch 4, wobei jede Seitenlasche (17, 17') der Vorrichtung eine Öffnung aufweist, die so positioniert ist, dass sie mit dem Gewindeloch (7, 7') und der Schraube (13, 13') ausgerichtet und konzentrisch ist.

6. Unterkiefervorverlagerungsvorrichtung nach Anspruch 5, wobei jede Öffnung der Vorrichtung so bemessen ist, dass ein Einstellwerkzeug eingeführt werden kann, und dadurch in die Schraube (13, 13') eingreifen und auf sie einwirken kann, jedoch zu klein ist, um das Herausziehen der Schraube (13, 13') durch die Öffnung zu ermöglichen, wodurch die Seitenlasche (17, 17') als Widerlager fungiert, das verhindert, dass die Schraube (13, 13') aus dem Körperabschnitt fällt.

7. Unterkiefervorverlagerungsvorrichtung gemäß Anspruch 1, wobei jeder Arm (3, 3') der Vorrichtung entweder in der oberen oder unteren Platte integriert ausgebildet und aus einem geeigneten Elastomermaterial hergestellt ist.

8. Unterkiefervorverlagerungsvorrichtung gemäß Anspruch 7, wobei jeder Arm der Vorrichtung in Bezug auf den anderen natürlich vorgespannt ist, mit einer maximalen Spreizung, bei der die Arme natürlich ruhen, wenn sie nicht durch das Einstellmittel beeinflusst werden.

9. Unterkiefervorverlagerungsvorrichtung gemäß Anspruch 1, wobei:
jeder Arm der Vorrichtung separat an entweder der oberen oder unteren Platte (1) hergestellt ist und entweder die obere oder untere Platte (1) einen jeweiligen Pfostenabschnitt für jeden Arm umfasst, der so profiliert ist, dass er einer Drehung um den Pfostenabschnitt widersteht und ein vorderes Ende jedes Arms ein Loch (33, 33') umfasst, das eine komplementäre Form mit dem Pfostenabschnitt aufweist und angepasst ist, um den Pfostenabschnitt in dem Loch (33, 33') aufzunehmen, wobei der Pfostenabschnitt in dem Loch (33, 33') aufgenommen wird, was eine natürliche Ruheposition für jeden Arm darstellt, wenn der Arm nicht durch das Einstellmittel beeinflusst wird; oder
jeder Arm der Vorrichtung entweder separat an der oberen Platte (15) oder der unteren Platte (1) gefertigt ist und entweder die obere Platte (15) oder die untere Platte (1) einen jeweiligen Pfostenabschnitt mit einem kreisförmigen Profil umfasst und ein vorderes Ende jedes Arms ein Loch umfasst, das angepasst ist, um den Pfostenabschnitt aufzunehmen und dem Arm zu ermöglichen, sich frei um seinen jeweiligen Pfostenabschnitt zu drehen, und der Körperabschnitt separates Vorspannmittel umfasst, das auf jeden jeweiligen Arm einwirkt und die Arme in ihrer maximal gespreizten Position hält, wenn auf jeden Arm nicht durch das Einstellmittel eingewirkt wird.

10. Unterkiefervorverlagerungsvorrichtung gemäß Anspruch 9, wobei das Vorspannmittel der Vorrichtung mindestens eine Schraubenfeder, eine Blattfeder oder eine Elastomerlasche ist, die entweder in der oberen Platte (15) oder der unteren Platte (1) enthalten ist, wobei die Elastomerlasche so angepasst ist, dass sie gegen einen jeweiligen Arm stößt.

11. Unterkiefervorverlagerungsvorrichtung gemäß einem der vorhergehenden Ansprüche, wobei entweder die obere oder die untere Platte (1) der Vorrichtung eine Öffnung oder ein Fenster aufweist, das mit Messinkrementanzeigen verbunden ist und die Messinkrementanzeigen eine aktuelle Position jedes Arms anzeigen, wenn der Arm durch das Einstellmittel in eine neue Position gezwungen wird.

12. Unterkiefervorverlagerungsvorrichtung gemäß Anspruch 11, wobei der Körperabschnitt der Vorrichtung elektronische Sensormittel umfasst, die dazu geeignet sind, die aktuelle Position jedes Arms zu erfassen und diese Positionsdaten an drahtlose Übertragungsmittel wie einen Bluetooth-Sender weiterzuleiten; und optional die Positionsdaten von den drahtlosen Übertragungsmitteln übertragen und von einer App auf einem tragbaren Computergerät verarbeitet werden.

13. Verfahren zum Anpassen der Unterkiefervorverlagerungsvorrichtung gemäß Anspruch 1, wobei das Verfahren Folgendes umfasst:
a. Auswählen einer geeigneten Unterkiefervorverlagerungsvorrichtung aus einer Auswahl von Unterkiefervorverlagerungsvorrichtungen in kleiner, mittlerer und großer Größe;
b. Einsetzen der Vorrichtung in den Mund eines Trägers;
c. Bestimmen der Passform und des Tragekomforts der Vorrichtung durch Abstützen jeder intraoralen Oberkieferanschlagfläche gegen eine extramaxilläre vordere Oberkieferanschlagfläche;
d. Empfangen von Feedback vom Träger zu Passform und Tragekomfort; und
e. Verwenden eines geeigneten Werkzeugs zum Eingreifen in das Einstellmittel, um jeden entsprechenden Arm zu einer Positionsänderung zu zwingen und dadurch jede hintere intraorale Oberkiefer-Anschlagfläche zu einer Positionsänderung zu veranlassen, um eine individuelle Passform des Geräts für den Träger bereitzustellen.

14. Verfahren nach Anspruch 13, wobei Schritt e. das Einführen eines geeigneten Werkzeugs in das Gewindeloch und dadurch das Einrasten einer Schraube des Einstellmittels und dessen Drehen umfasst, um jeden jeweiligen Arm zu zwingen, seine Position zu ändern, und dadurch zu bewirken, dass jede hintere intraorale Oberkiefer-Anschlagfläche ihre Position ändert, um eine individuelle Passform des Geräts für den Träger bereitzustellen.

15. Verfahren nach Anspruch 13 oder Anspruch 14, wobei entweder die obere oder untere Platte (1) des Geräts zusätzlich eine Öffnung oder ein Fenster aufweist, das mit Messinkrementanzeigen verbunden ist und die Messinkrementanzeigen eine aktuelle Position jedes Arms anzeigen, wenn der Arm unter dem Einfluss der Schraube in eine neue Position gezwungen wird, wobei das Verfahren zusätzlich die Verwendung der Öffnung oder des Fensters und der zugehörigen Messinkrementanzeigen umfasst, um die bequemste Einstellung für den jeweiligen Träger zu notieren, und optional wobei der Körperabschnitt des Geräts ferner elektronische Sensormittel umfasst, die dazu geeignet sind, die aktuelle Position jedes Arms zu erfassen und diese Positionsdaten an drahtlose Übertragungsmittel wie einen Bluetooth-Sender weiterzuleiten, und wobei die Positionsdaten durch die drahtlosen Übertragungsmittel übertragbar und durch eine App auf einem tragbaren Computergerät verarbeitbar sind, wobei das Verfahren ferner die Verwendung der Sensormittel und drahtlosen Übertragungsmittel umfasst, um die Positionsdaten jedes Arms in einer App auf einem tragbaren Computergerät zu übertragen, um die optimale Position jedes Arms dem Träger zuzuordnen.

## Revendications

1. Dispositif d'avancement mandibulaire comportant :
une partie intraorale comportant une partie corps, la partie corps comportant des plaques inférieure (1) et supérieure (15), la plaque inférieure (1) étant conçue pour recevoir une paire de bras (3, 3') opposés, qui s'étendent vers l'arrière et vers l'extérieur à partir de la plaque inférieure (1) dans une cavité orale lorsque le dispositif est porté, chaque bras comportant une surface de butée maxillaire intraorale postérieure, et une partie extraorale,
**caractérisé en ce que**
le dispositif d'avancement mandibulaire comporte un moyen de réglage disposé de chaque côté de la plaque inférieure (1), le moyen de réglage étant conçu pour agir sur un bras respectif pour déplacer le bras respectif en direction de son bras opposé, de sorte que la surface de butée maxillaire intraorale postérieure du bras respectif soit déplacée vers une nouvelle position.

2. Dispositif d'avancement mandibulaire selon la revendication 1 dans lequel le moyen de réglage comprend des vis (13, 13'), et dans lequel chaque côté de la plaque inférieure (1) du dispositif comporte un trou fileté (7, 7'), adjacent à un bras respectif, de sorte que le trou fileté (7, 7') s'étende à partir du bras respectif en direction d'un bord de la plaque inférieure (1), et dans lequel chaque trou (7, 7') est conçu pour recevoir une vis du moyen de réglage qui peut être vissée dans le trou fileté (7, 7'), et lorsque la vis (13, 13') a été suffisamment vissée dans ledit trou fileté (7, 7'), une extrémité interne de la vis (13, 13') entre en contact avec son bras respectif et tout en tournant davantage la vis (13, 13') force le bras respectif à se déplacer en direction de son bras opposé de sorte que la surface de butée maxillaire intraorale postérieure du bras respectif soit déplacée vers une nouvelle position.

3. Dispositif d'avancement mandibulaire selon la revendication 2 dans lequel :
chaque trou fileté (7, 7') dans la plaque inférieure (1) du dispositif est évidé vers l'arrière à partir du bord et une ouverture du trou fileté (7, 7') est respectivement située à l'intérieur d'une fente verticale (9, 9') ; et éventuellement
la plaque supérieure (15) du dispositif est conçue pour recouvrir la plaque inférieure (1) et s'assembler conjointement avec la plaque inférieure (1) pour former la partie corps et encapsuler une partie de chacun des bras à l'intérieur de la partie corps.

4. Dispositif d'avancement mandibulaire selon la revendication 3 dans lequel la plaque supérieure (15) du dispositif comporte des languettes latérales (17, 17') qui s'étendent sensiblement verticalement vers le bas et sont alignées avec et conçues pour être insérées dans, et se conformer à la forme de, la fente verticale (9, 9'), recouvrant par conséquent l'ouverture du trou fileté (7, 7') et de la vis (13, 13') et formant la partie corps avec une surface externe sensiblement lisse.

5. Dispositif d'avancement mandibulaire selon la revendication 4 dans lequel chaque languette latérale (17, 17') du dispositif comporte une ouverture qui est positionnée de sorte qu'elle s'aligne avec, et est concentrique avec, le trou fileté (7, 7') et la vis (13, 13').

6. Dispositif d'avancement mandibulaire selon la revendication 5 dans lequel chaque ouverture du dispositif est dimensionnée de sorte qu'un outil de réglage puisse être inséré, et peut ainsi venir en prise avec et agir sur la vis (13, 13'), mais est trop petite pour permettre à la vis (13, 13') d'être retirée à travers l'ouverture, amenant ainsi la languette latérale (17, 17') à agir comme une butée qui empêche la vis (13, 13') de tomber de la partie corps.

7. Dispositif d'avancement mandibulaire selon la revendication 1 dans lequel chaque bras (3, 3') du dispositif est formé d'un seul tenant dans la plaque supérieure ou inférieure et fabriqué à partir d'un matériau élastomère adapté.

8. Dispositif d'avancement mandibulaire selon la revendication 7 dans lequel chaque bras du dispositif est naturellement sollicité par rapport à l'autre avec un écart maximal qui est celui du repos naturel des bras lorsqu'ils ne sont pas actionnés par le moyen de réglage.

9. Dispositif d'avancement mandibulaire selon la revendication 1 dans lequel :
chaque bras du dispositif est fabriqué séparément de la plaque supérieure ou inférieure (1), et la plaque supérieure ou inférieure (1) comporte une partie montant respective pour chaque bras qui est profilée pour résister à une rotation autour de la partie montant, et une extrémité antérieure de chaque bras comporte un trou (33, 33') qui présente une forme complémentaire avec, et est conçu pour recevoir, la partie montant à l'intérieur dudit trou (33, 33'), la partie montant étant reçue à l'intérieur dudit trou (33, 33') étant une position de repos naturel pour chaque bras lorsque le bras n'est pas actionné par les moyens de réglage ; ou
chaque bras du dispositif est fabriqué séparément de la plaque supérieure (15) ou de la plaque inférieure (1), et la plaque supérieure (15) ou la plaque inférieure (1) comporte une partie montant respective avec un profil circulaire, et une extrémité antérieure de chaque bras comporte un trou qui est conçu pour recevoir la partie montant et permettre au bras de tourner librement autour de sa partie montant respective, et la partie corps comporte des moyens de sollicitation distincts qui agissent sur chaque bras respectif et maintiennent les bras au niveau de leur position d'écartement maximal lorsque chaque bras n'est pas actionné par le moyen de réglage.

10. Dispositif d'avancement mandibulaire selon la revendication 9 dans lequel les moyens de sollicitation du dispositif sont au moins l'un d'un ressort hélicoïdal, d'un ressort à lames, ou d'une languette élastomère qui est incluse dans la plaque supérieure (15) ou dans la plaque inférieure (1), la languette élastomère étant conçue pour empiéter sur un bras respectif.

11. Dispositif d'avancement mandibulaire tel que défini dans une quelconque revendication précédente dans lequel soit la plaque supérieure soit la plaque inférieure (1) du dispositif comporte une ouverture, ou fenêtre, qui est associée à des indicateurs d'incrément de mesure, et lesdits indicateurs d'incrément de mesure indiquent une position actuelle de chaque bras lorsque le bras est forcé vers une nouvelle position par le moyen de réglage.

12. Dispositif d'avancement mandibulaire selon la revendication 11 dans lequel la partie corps du dispositif comporte un moyen de capteur électronique qui est conçu pour détecter la position actuelle de chaque bras et introduire ces données de position dans des moyens de transmission sans fil tels qu'un émetteur/récepteur Bluetooth ; et éventuellement les données de position sont transmises par les moyens de transmission sans fil et sont traitées par une application sur un dispositif de calcul portatif.

13. Procédé de réglage du dispositif d'avancement mandibulaire selon la revendication 1, le procédé comportant :
a. la sélection d'un dispositif d'avancement mandibulaire approprié à partir d'une sélection de petits, moyens et grands dispositifs d'avancement mandibulaire ;
b. l'insertion du dispositif dans la bouche d'un utilisateur ;
c. la détermination d'un ajustement et d'un confort du dispositif par consolidation de chaque surface de butée maxillaire intraorale contre une surface de butée maxillaire antérieure extramaxillaire ;
d. la réception d'un retour d'informations du porteur sur l'ajustement et le confort ; et
e. l'utilisation d'un outil approprié pour venir en prise avec le moyen de réglage pour forcer chaque bras respectif à changer de position et amenant ainsi chaque surface de butée maxillaire intraorale postérieure à changer de position pour fournir un ajustement personnalisé du dispositif pour le porteur.

14. Procédé selon la revendication 13 dans lequel l'étape e. comprend l'utilisation d'un outil approprié destiné à être inséré dans le trou fileté et entrant ainsi en prise avec une vis du moyen de réglage et tournant celle-ci pour forcer chaque bras respectif à changer de position et amenant ainsi chaque surface de butée maxillaire intraorale postérieure à changer de position pour fournir un ajustement personnalisé du dispositif pour le porteur.

15. Procédé selon la revendication 13 ou la revendication 14 dans lequel la plaque supérieure ou inférieure (1) du dispositif comporte en outre une ouverture, ou fenêtre, qui est associée à des indicateurs d'incrément de mesure, et lesdits indicateurs d'incrément de mesure indiquent une position actuelle de chaque bras lorsque le bras est forcé vers une nouvelle position sous l'influence de la vis, ledit procédé comportant en outre l'utilisation de l'ouverture, ou de la fenêtre, et de ses indicateurs d'incrément de mesure associés pour prendre note de l'ajustement le plus confortable pour le porteur en question, et éventuellement
dans lequel la partie corps du dispositif comporte en outre des moyens de capteur électronique qui sont conçus pour détecter la position actuelle de chaque bras et introduire ces données de position dans des moyens de transmission sans fil tels qu'un émetteur/récepteur Bluetooth, et dans lequel les données de position peuvent être transmises par les moyens de transmission sans fil et peuvent être traitées par une application sur un dispositif de calcul portatif, ledit procédé comportant en outre l'utilisation des moyens de capteur et des moyens de transmission sans fil pour transmettre les données de position de chaque bras dans une application sur un dispositif de calcul portatif pour associer la position optimale de chaque bras au porteur.
